Europäisches Patentamt

**European Patent Office**

**Office européen des brevets**

(19)

(11) Publication number: **0 026 927**
**A1**

(12) # EUROPEAN PATENT APPLICATION

(21) Application number: **80106000.5**

(22) Date of filing: **03.10.80**

(51) Int. Cl.³: **C 07 J 9/00**

(30) Priority: **04.10.79 US 81956**

(43) Date of publication of application: **15.04.81**
**Bulletin 81/15**

(84) Designated Contracting States: **BE CH DE FR GB LI NL**

(71) Applicant: **EASTMAN KODAK COMPANY, 343 State Street, Rochester, New York 14650 (US)**

(72) Inventor: **Foster, Charles Howard, P.O. Box 511, Kingsport Tennessee (US)**

(74) Representative: **Brandes, Jürgen, Dipl.-Chem. Dr. et al, Thierschstrasse 8, D-8000 München 22 (DE)**

(54) **Process for dehydrogenation of 3-beta-hydroxy steroids.**

(57)   This invention relates to the dehydrogenation of 3-β--hydroxy steroids, such as a mixture of soy sterols, to form the corresponding Δ⁴-3-keto derivatives of the steroids. An activated non-pyrophoric nickel catalyst is used for the reaction and a diaryl ketone is used in the reaction as a hydrogen acceptor.

-1-

## PROCESS FOR DEHYDROGENATION OF 3-β-HYDROXY STEROIDS

This invention relates to the dehydrogenation of 3-β-hydroxy steroids, such as a mixture of soy sterols to form the corresponding $\Delta^4$-3-keto derivatives of the steroids using an activated non-pyrophoric nickel as a catalyst and a diaryl ketone as a hydrogen acceptor.

The naturally occurring phytosterol components of vegetable oils, such as soy oils are composed of mixtures of phytosterols. These phytosterols can be used in the preparation of pharmaceuticals, such as progesterone, which also can be used to prepare other steroids, such as cortisone. The first step in the preparation of progesterone from soy sterols involves the dehydrogenation of the sterols to form the $\Delta^4$-3-keto derivatives. The 3β-OH of the sterol is dehydrogenated to a ketone with rearrangement of the $\Delta^5$ double bond into conjugation with the ketone. Catalytic dehydrogenation is an economical method for this oxidation-rearrangement process. Catalytic dehydrogenation provides a process for dehydrogenation of $\Delta^5$-sterols to ketones with a simultaneous shift of the $\Delta^5$ double bond to the $\Delta^4$ position in conjugation with the carbonyl group. Raney nickel has been used for this reaction, as disclosed in Chakravarti, Chakravarti, and Mitra, Nature, 193, 1071 (1962) or in the presence of a hydrogen acceptor as disclosed in E. C. Kleiderer, and E. C. Kornfeld, J. Org. Chem., 13, 455 (1948) and Kleiderer, Rice, Conquest and Williams, U.S. Dept. of Commerce, Office of the Publication Board, Report PB 981, 1945.

The reaction disclosed by Kleiderer et al was carried out with Raney nickel as a catalyst and cyclohexanone as a hydrogen acceptor. The reaction was carried out for a period of 24 hours and the yield of product varied from 30 to 80 percent.

In accordance with this invention product yields of 80 percent and higher can be obtained in much shorter reaction times. In this invention 3-β-hydroxy steriods, such as mixtures of soy sterols, are dehydrogenated to form the corresponding $\Delta^4$-3-keto derivatives using an activated non-pyrophoric nickel, such as Raney nickel, as a catalyst and using a diaryl ketone as a hydrogen acceptor. The specific examples that illustrate this invention show that product yields of 82 and 85 percent can be obtained in reaction periods of only 2 and 2 1/2 hours by using this invention.

The activated non-pyrophoric nickel catalyst can be a Raney nickel catalyst, preferably an activated non-pyrophoric nickel powder such as that supplied by Ventron Corp. The dehydrogenation reaction is carried out in the presence of a diaryl ketone hydrogen acceptor. The amount of diaryl ketone employed in the dehydrogenation is from 1 mole of ketone per mole of steroid or soy sterols up to 3 moles of ketone per mole of sterioid or soy sterols. The rate of the dehydrogenation is reduced as the amount of ketone is increased. A small amount of base (e.g. $K_2CO_3$) can be used in the reaction to minimize dehydration of the steroids at the elevated temperature. The amount of base employed is 1/30, by weight, based on the weight of steroids or soy sterols. The hydrogen acceptors for example can be, benzophenone or substituted benzophenones. The dehydrogenation is preferably carried out at elevated temperatures, such as 150°C. to 350°C., preferably 200°C.-300°C., and most preferably 240-260°C. At temperatures lower than 150°C., the dehydrogenation reaction is too slow and above 350°C. there is some decomposition of the steroids. Use of benzophenone allows the reaction to be carried out at atmospheric pressure in a reasonable reaction rate. The period of time used for the dehydrogenation depends on the temperature used. However, at temperatures of 200°C.-300°C. the reaction is substantially completed after 8 hours.

The amount of catalyst employed varies with the amount of steroid used and the speed of reaction desired for the dehydrogenation reaction. The amount of catalyst used can be equal to 20 to 40 percent, preferably 25 to 30 percent, based on the weight of the steroids. It is a unique advantage of this process that a solvent is not necessary. However, a solvent can be used if desired.

EXAMPLE 1

Preparation of $\Delta^4$-3-keto derivatives of soy sterols was carried out using 30.0 g of soy sterols ( 22% stigmasterol, 78% sitosterol/campesterol), 15 g of benzophenone and 12 g of activated non-pyrophoric nickel catalyst (Ventron). The mixture was heated at 260° for 2 hrs. in a stirred round bottom flask. The mixture was cooled to room temperature. Assay of the product showed it to be 85% $\Delta^4$-3-ketosteroids. The product was isolated by dissolving in $CH_2Cl_2$, filtering off the catalyst and then removing the $CH_2Cl_2$ and benzophenone/benzohydrol/diphenylmethane under vacuum. The $\Delta^4$-3-keto derivatives of soy sterols are then reacted by ozonolysis to form 3-ketodinor-4-cholen-22-aldehyde which can be isolated from the other $\Delta^4$-3-keto derivatives of soy sterols by either chromatography or by treatment with sodium bisulfite and extraction with a suitable organic solvent such as toluene.

EXAMPLE 2

Stigmasterol (25.0 g), benzophenone (12.5 g), activated non-pyrophoric nickel catalyst (10 g, Ventron Corp.), and 1.0 g of $K_2CO_3$ were heated at 260° for 2-1/2 hrs. Assay of the reaction mixture showed at least 82% 4,22-stigmastadien-3-one with only minor amounts of by-products. The mixture was cooled and dissolved in ethyl acetate, filtered and concentrated in vaccuo. Benzophenone and its reduction products were distilled off at 150-200° (5-10 torr). The residue (25.0 g) was recrystallized from hexane to give 12.5 g of pure 4,22-stigmastadiene-3-one (mp 121-123°).

EXAMPLE 3

Cholesterol (20 g), benzophenone (12.5 g), activated non-pyrophoric nickel catalyst (10 g, Ventron Corp.), and 1.0 g of $K_2CO_3$ were heated at 260° for 2-1/2 hrs. Assay of the reaction mixture showed at least 82% 4-cholesten-3-one with only minor amounts of by-products. The mixture was cooled and dissolved in ethyl acetate, filtered and concentrated in vaccuo. Benzophenone and its reduction products were distilled off at 150-200°C (5-10 torr).

EXAMPLE 4

Sitosterol (25.0 g), benzophenone (12.5 g), activated non-pyrophoric nickel catalyst (10 g, Ventron Corp.), and 1.0 g of $K_2CO_3$ were heated at 260° for 2-1/2 hrs. The mixture was cooled and dissolved in ethyl acetate, filtered and concentrated in vaccuo. Benzophenone and its reduction products were distilled off at 150-200° (5-10 torr).

The process of the present invention provides and improved method for the dehydrogenation of 3-β-hydroxy steriods to $\Delta^4$-3-ketosteroids. The $\Delta^4$-3-ketosteroids can be used to provide starting materials for preparation of valuable steroids such as the cortical steroids.

Claims:

1. A process for dehydrogenating 3-β-hydroxy steroids to form the corresponding $\Delta^4$-3-keto derivatives characterized by using an activated non-pyrophoric nickel as a catalyst and a diaryl ketone as a hydrogen acceptor.

2. A process according to Claim 1 wherein Raney nickel is the catalyst.

3. A process according to Claim 1 wherein benzophenone is the hydrogen acceptor.

**European Patent Office**

# EUROPEAN SEARCH REPORT

## DOCUMENTS CONSIDERED TO BE RELEVANT

| Category | Citation of document with indication, where appropriate, of relevant passages | Relevant to claim |
|---|---|---|
| D | JOURNAL OF ORGANIC CHEMISTRY, vol. XIII, no. 3, May 1948, pages 455-458<br>Washington D.C., U.S.A.<br>E.C. KLEIDERER et al.: "Raney nickel as an organic oxidation-reduction catalyst"<br><br>* Whole article *<br><br>-- | 1 |
| | CHEMICAL ABSTRACTS, vol. 48, no. 9, 14th May 1954, page 5202, column 2, no. 5202e<br>Columbus, Ohio, U.S.A.<br>J. ROMO: "Catalytic oxidations with Raney nickel"<br><br>& BOL. INST. QUIM. UNIV. NACL. AUTON. MEX. 4, 91-100, 1952<br><br>* Abstract *<br><br>-- | 1 |
| A | US - A - 2 890 226 (ARTHUR R. HANZE)<br><br>* Whole patent *<br><br>-- | 1 |
| A | RECUEIL DES TRAVAUX CHIMIQUES DES PAYS BAS, vol. 56, no. 1, January 1937, pages 137-144<br>Amsterdam, NL.<br>R.V. OPPENAUER: "Eine Methode der Dehydrierung von sekundären Alkoholen zu Ketonen. I. Zur Herstellung von Sterinketonen und Sexualhormonen"<br><br>* Whole article *<br><br>---- | 1 |

### CLASSIFICATION OF THE APPLICATION (Int. Cl.³)

C 07 J 9/00

### TECHNICAL FIELDS SEARCHED (Int. Cl.³)

C 07 J 9/00

### CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

The present search report has been drawn up for all claims

| Place of search | Date of completion of the search | Examiner |
|---|---|---|
| The Hague | 11-12-1980 | HENRY |

EPO Form 1503.1 06.78